# EUROPEAN PATENT SPECIFICATION

(11) **EP 3 487 380 B1**
(45) Date of publication and mention of the grant of the patent: **14.04.2021**
(21) Application number: 17740807.7
(22) Date of filing: 22.06.2017
(51) Int. Cl.: A61B 1/12

(54) **INSERT CONFIGURED FOR BEING ARRANGED IN A DEVICE FOR CLEANING MEDICAL INSTRUMENTS**
EINSATZ ZUR ANORDNUNG IN EINER VORRICHTUNG FÜR DIE REINIGUNG VON MEDIZINISCHEN INSTRUMENTEN
INSERT CONÇU POUR ÊTRE DISPOSÉ DANS UN DISPOSITIF DE NETTOYAGE D'INSTRUMENTS MÉDICAUX

(30) Priority: 21.07.2016 NL 2017202
(43) Date of publication of application: 29.05.2019
(73) Proprietor: P.M.T. Partners Medische Techniek B.V., 2952 BD Alblasserdam (NL)
(72) Inventor: SCHOUWENBURG, John, 2952 BD Alblasserdam (NL); SCHOUWENBURG, Marco, 2952 BD Alblasserdam (NL)
(74) Representative: Algemeen Octrooi- en Merkenbureau B.V.
(86) International application number: PCT/NL2017/050416
(87) International publication number: WO 2018/016942

(56) References cited:
- EP-A1- 1 785 150
- EP-A1- 2 837 353
- WO-A1-2016/013275
- JP-A- H1 199 121

## Description

### FIELD OF THE INVENTION

The present invention relates to an insert configured for being arranged in a device for cleaning medical instruments, preferably by ultrasonic cleaning, the insert being arranged for receiving one or more medical instruments to be cleaned in the cleaning device. The invention also relates to a cleaning device for receiving such an insert and an assembly of the aforementioned insert and the aforementioned cleaning device.

### BACKGROUND OF THE INVENTION

Various cleaning devices are known in the art for cleaning medical instruments, such as the cleaning devices provided by P.M.T. Partners Medische Techniek B.V. of the Netherlands. Such cleaning devices are particularly suited for cleaning endoscopic instruments with appropriate cleaning fluids, wherein even the hollow parts of the endoscopic instruments can be properly cleaned.

A problem with the known cleaning devices is that such devices are usually suitable for cleaning only one type of medical instrument, i.e. such cleaning devices are normally purpose-built for this type of instrument, such as the *da Vinci*® Si endoscopic instrument sold by Intuitive Surgical, Inc., of the United States of America. Thus, the range of application of such a cleaning device could be improved.

EP 2 837 353 B1 discloses an ultrasonic cleaning device for cleaning medical instruments and an insert to be used in such an ultrasonic cleaning device. However, the insert is relatively heavy, has a complex construction and is cumbersome to use. In particular EP 2 837 353 B1 discloses an insert according to the preamble of claim 1.

EP 1 785 150 A1 discloses an apparatus for washing and disinfecting endoscopes, as well as a method for examining water leakage.

An object of the invention is thus to provide a cleaning device being capable of cleaning a wide range of medical instruments, as well as an insert for use with such a cleaning device, wherein the insert is less heavy, has a simpler construction and is easier to use.

### SUMMARY OF THE INVENTION

Hereto, according to the invention an insert is provided configured for being arranged in a device for cleaning medical instruments, the insert being arranged for receiving one or more medical instruments to be cleaned in the cleaning device, wherein the one or more medical instruments are provided with one or more operating elements for moving one or more moveable parts of the medical instruments, the insert comprising operating means for operating the operating elements during cleaning, the operating means being arranged for connection with a motor for driving the operating means during cleaning.

Due to the provision an (exchangeable) insert a wide variety of medical devices can be cleaned in the cleaning device. I.e. an insert can be specifically designed or arranged for the specific medical instrument to be cleaned, such as the *da Vinci*® *Xi* endoscopic instrument provided by Intuitive Surgical, Inc., as mentioned before. This also holds for the operating means to operate the operating elements during cleaning.

According to the invention, the cleaning device comprises the motor for driving the operating means. This allows for a much simpler construction of the insert and the insert will also become less heavy.

According to the invention, the insert is provided with insert-side coupling means driveably coupled to the operating means, the insert-side coupling means being arranged for being detachably coupled to machine-side coupling means comprised by the cleaning device, the machine-side coupling means being driveably coupled to the motor, such that, during use, the operating means of the insert can be driven by the motor. Each individual insert design may comprise its own specific operating means construction to drive the operating elements, with the insert-side coupling means being detachably connected the machine-side coupling means to transfer movement provided by the cleaning device motor to the operating means, at all times ensuring proper cleaning of the respective medical device(s).

An embodiment relates to an aforementioned insert, comprising one or more mounting blocks for mounting the one or more medical instruments. Such mounting blocks, preferably made of plastic, securely arrange the medical instrument in the insert and position the medical instrument for being properly cleaned.

An embodiment relates to an aforementioned insert, wherein the one or more mounting blocks comprise one or more clamps for properly connecting and positioning the one or more medical instruments with respect to the operating means during use. Such clamps facilitate placement and easy removal of the medical instruments in/on the mounting block. At the same time the clamps ensure a proper connecting between the operating elements of the medical instrument and the operating means comprised by the insert.

An embodiment relates to an aforementioned insert, wherein the operating means for operating the operating elements during cleaning are configured for operating the operating elements in a non-simultaneous manner during use. Thus, it is ensured that proper cleaning of the medical instrument takes place without operating each individual operating element at the same time, which could hamper cleaning or even lead to damaging of the medical instrument.

An embodiment relates to an aforementioned insert, wherein the insert is shaped like a tray. Such a tray can be easily handled by a person and has holes or open spaces in the sidewalls thereof to facilitate the entrance of cleaning fluid into the interior of the tray where the medical instruments are arranged.

Preferably, the insert in general is relatively flat to improve handling. I.e. its height (when seen in sideways direction during use) is for instance at most 20% or even at most 10% or even at most 5% of its length and or width. The height of the insert is preferably determined by the size of the medical instruments, with the medical instruments preferably being arranged in lengthwise or widthwise direction during use. Thus the height of the insert could be 105%-120%, such as approximately 110% of the height of the medical instrument during use. For instance, in case of an endoscopic instrument the lumen may extend in lengthwise direction with the handheld part of the endoscopic instrument then dictating the height of the insert.

An embodiment relates to an aforementioned insert, wherein the insert comprises three compartments, a central compartment, such as for comprising the insert-side coupling means, and two side compartments arranged for comprising the one or more medical instruments. This provides an optimal balance between insert (medical instrument) capacity and ease of construction of the insert-side coupling means and the operating means.

An embodiment relates to an aforementioned insert, wherein the one or more mounting blocks are arranged in one or more rows of mounting blocks, such that, during cleaning, the medical instruments are arranged in one or more rows of instruments and a reciprocating member is provided for reciprocating past the operating means of a row of mounting blocks. When for instance an insert-design with a central compartment and two side compartments is used, two rows of instruments, such as rows of 2-8, preferably 4-6, instruments each can be driven with a single reciprocating movement.

An embodiment relates to an aforementioned insert, comprising connection means for connecting one or more pressurized channels to the one or more medical instruments. Preferably, per row a cable with an amount of pressure channels equal to the amount of instruments in the row is provided. The cable is preferably arranged for detachable connection to a cable connection point on the insert/tray. At the cable connection point the insert is preferably provided with distribution means providing individual pressurized channels to each of the medical instruments (wherein each respective pressurized channel connects to the respective pressurized channel comprised by the cable via the distribution means).

Another aspect of the invention concerns a device for, preferably ultrasonic, cleaning medical instruments, arranged for receiving an aforementioned insert.

According to the invention, the cleaning device comprises the motor for driving the operating means, allowing the insert to become much lighter and easier to construct.

According to the invention, the cleaning device comprises machine-side coupling means for being detachably coupled to the insert-side coupling means, the machine-side coupling means being driveably coupled to the motor, such that, during use, the operating means of the insert can be driven by the motor.

An embodiment relates to an aforementioned device, wherein the cleaning device comprises a reception space for receiving the insert at the top side of the cleaning device. Thus, the reception space is easy to reach for a person desiring to arrange the exchangeable insert in the cleaning device.

An embodiment relates to an aforementioned device, wherein the reception space is configured for containing a cleaning fluid for cleaning the one or more medical instruments during use. The tray/insert of course has to be able to resist corrosion, et cetera, caused by the cleaning fluid, which usually comprises one or more chemical agents. The insert also has to be provided with holes or open spaces in its sidewalls to facilitate entry of the cleaning fluid into the interior of the insert and towards the medical instruments to be cleaned. Preferably, the insert is open at its top side (i.e. in its position of use) to allow the operator of the device to see what is happening "inside" the insert. The device then is preferably provided with a lid, such as a transparent lid, to prevent the operator from coming onto contact with the cleaning fluid during operation of the cleaning device.

An embodiment relates to an aforementioned device, wherein the reception space is provided with means for generating ultrasonic sound waves in the cleaning fluid. Such ultrasonic sound waves allow a very high degree of cleaning, even allowing cleaning of the hard-to-reach parts of the medical instruments.

An embodiment relates to an aforementioned device, wherein the means for generating ultrasonic sound waves comprise piezoelectric elements.

An embodiment relates to an aforementioned device, wherein the means for generating ultrasonic sound waves are arranged underneath a bottom side of the reception space, the means being connected to the bottom side in such a way, that the ultrasonic sound waves are transferred to the cleaning fluid by the bottom side. Thus, the means are themselves shielded from the cleaning fluid, but allow efficient transfer of sound waves via the bottom side of the reception space. This bottom side of course has to be made of a suitable material to be able to achieve this.

Another aspect of the invention concerns an assembly of an aforementioned insert and an aforementioned cleaning device for cleaning medical instruments, wherein the cleaning device comprises the motor for driving the operating means, wherein the insert is provided with insert-side coupling means driveably coupled to the operating means, the insert-side coupling means being detachably coupled to machine-side coupling means comprised by the cleaning device, the machine-side coupling means being driveably coupled to the motor, such that, during use, the operating means of the insert can be driven by the motor.

### BRIEF DESCRIPTION OF THE DRAWINGS

The present invention will be explained hereafter with reference to exemplary embodiments of an insert and a cleaning device according to the invention and with reference to the drawings. Therein:
Figure 1 shows a perspective view of an exemplary embodiment of an insert according to the invention;
Figure 2 shows a pair of endoscopic instruments;
Figure 3 shows a perspective view of an exemplary embodiment of a cleaning device according to the invention;
Figure 4 shows a cut-away view of a lower region of the reception space;
Figure 5 shows a perspective view of the operating means;
Figure 6 shows an exemplary configuration of protrusions on an elongated member to drive the operating means in a non-simultaneous manner; and Figure 7 shows a detailed view of a mounting block.

### DETAILED DESCRIPTION

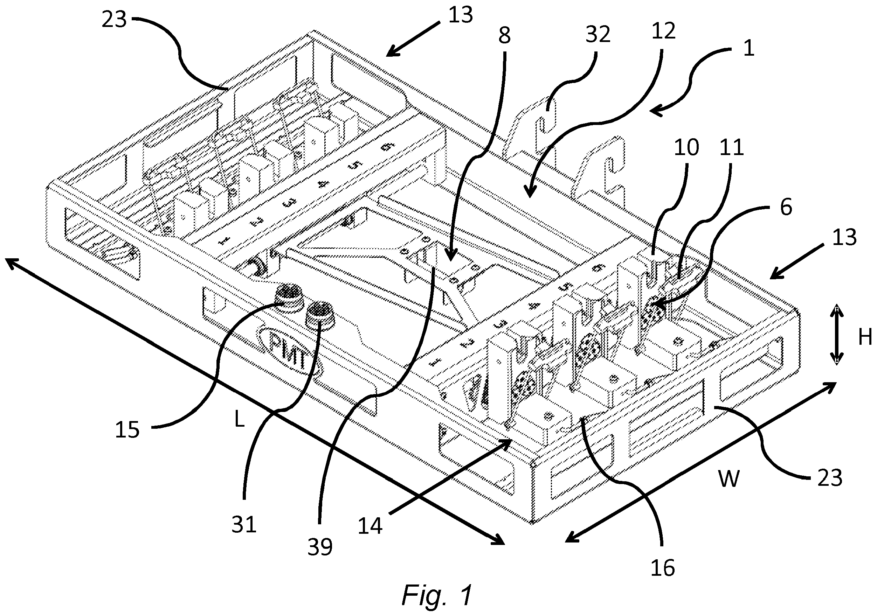

Figure 1 shows an insert 1 (depicted in its position of use) having a length L, a width W and a height H, configured for being arranged in a device for cleaning medical instruments (not shown in figure 1). The length L could amount to for instance to 0.5 - 1.5 m. The height H could be for instance 0.1 - 0.3 m. The width W could be for instance 0.25 - 0.75 m. Of course, these dimensions depend on the type and amount of medical instruments to be cleaned. The insert 1 preferably is shaped as a relatively flat tray or crate and is preferably made of a lightweight material, such as plastic, able to resist multiple consecutive exposures to the cleaning fluid.

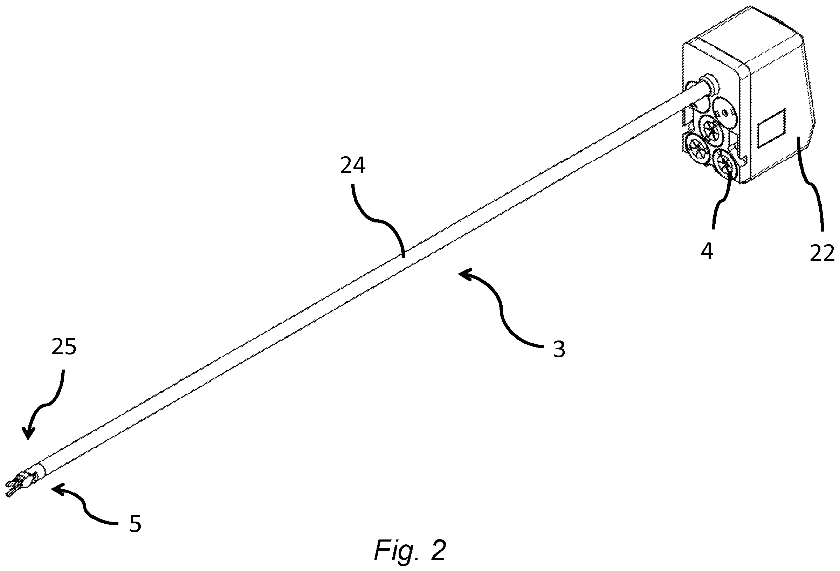

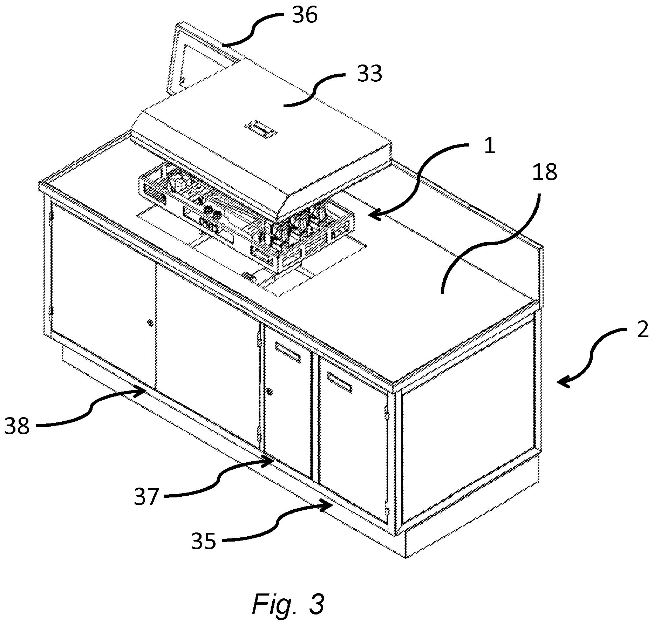

The insert 1 as shown is arranged for receiving one or more medical instruments 3 (please refer to figure 2) to be cleaned in the cleaning device 2 (please refer to figure 3), such as endoscopic instruments 3. As shown in figure 2, the one or more medical instruments 3 are provided with one or more operating elements 4 in the form of wheels or knobs for moving one or more moveable parts 5 of the medical instruments 3, such as the distal part/head 25 of an endoscopic instrument 3. The endoscopic instruments 3 are preferably arranged with their holder/handheld part 22 near the sidewalls 23 delimiting the insert 1 in lengthwise (L) direction with the lumen 24 of the endoscopic instrument 3 (horizontally) pointing inwards in lengthwise (L) direction. When cleaning the *da Vinci*® *Xi* endoscopic instrument, the holder part 22 and lumen 24 will be at a perpendicular angle, due to the design of the instrument. The insert 1 further comprises a pair of suspension hooks 32 to properly suspend the insert 1 in the cleaning fluid during use.

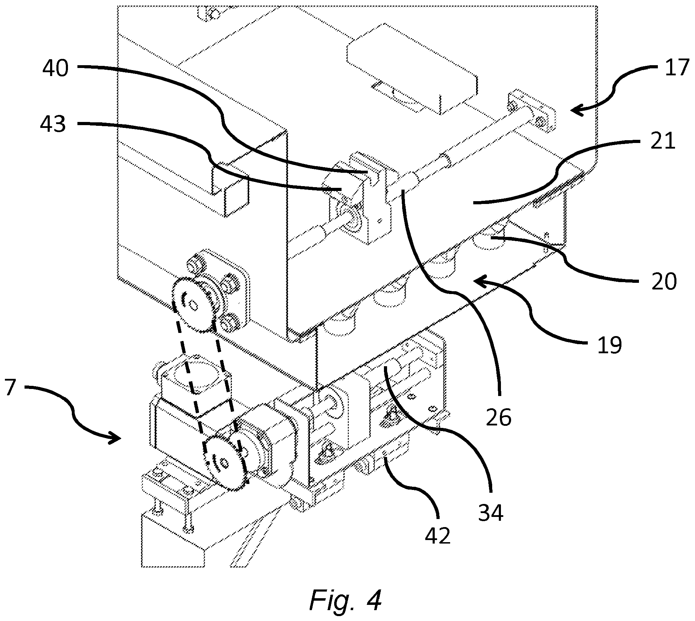

As shown in figure 1, the insert 1 comprises operating means 6 for operating the operating elements 4 during cleaning, in such a way, that the one or more moveable parts 5 are moved to facilitate thorough cleaning. The cleaning device further comprises a motor (not shown) for driving the operating means 6. The insert 1 is provided with insert-side coupling means 8 driveably coupled to the operating means 6. The insert-side coupling means 8 are arranged for being detachably coupled to machine-side coupling means (please refer to figure 4) comprised by the cleaning device 2. For instance by means of a pin 39 comprised by the insert-side coupling means 8. The machine-side coupling means 9 are then driveably coupled to the motor, such that, during use, the operating means 6 of the insert 1 can be driven (such as moved back and forth in a reciprocating manner) by the motor 7. The machine-side coupling means 9 may comprise a recess 40 to engage the pin. To facilitate coupling the pin 39 may be arranged to be vertically moveable and the recess 40 may be flanked by two tilted/angled upper walls or faces 43 to guide the pin 39 towards the recess 40 during a reciprocating movement of the machine-side coupling means 9.

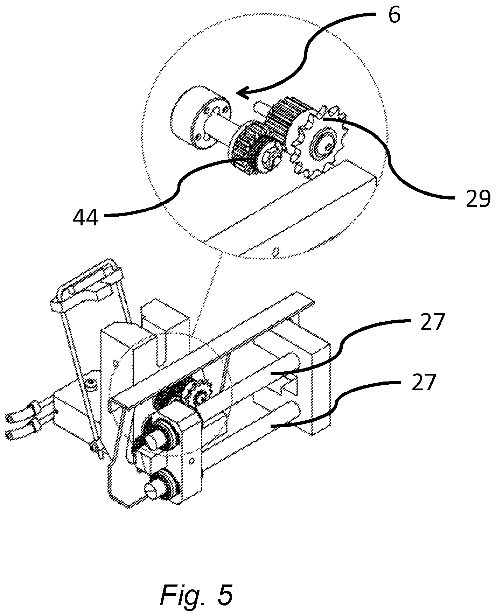

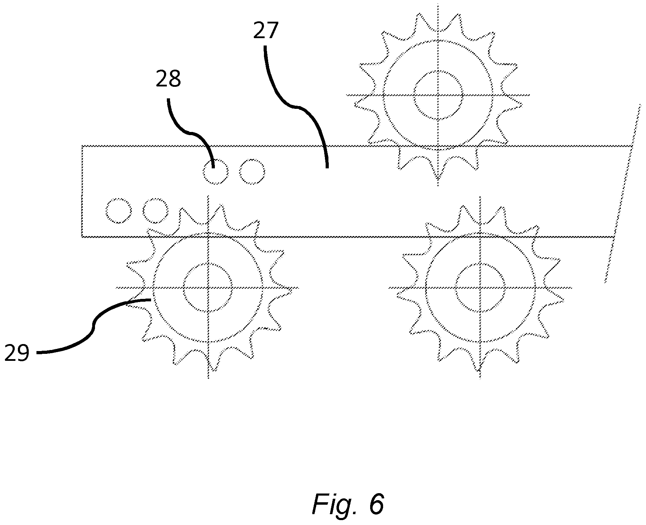

The insert 1 also comprises one or more mounting blocks 10 for mounting the one or more medical instruments 3. The one or more mounting blocks 10 each comprise one or more clamps 11 for properly connecting the medical instruments 3 to the mounting block 10 and positioning the one or more medical instruments 3 with respect to the operating means 6 during use. Especially when the *da Vinci*® *Xi* endoscopic instrument is to be cleaned, with its holder part 22 then being connected to the mounting block 10 in a horizontal manner (i.e. with the holder part 22 essentially extending vertically and the lumen 24 extending horizontally) it is important to establish a proper connection. The operating means 6 for operating the operating elements 4 during cleaning are configured for operating the operating elements 4 in a non-simultaneous manner during use. Therein, the machine-side coupling means 9 (please refer to figure 4) are caused to make a reciprocating movement due to a spindle 26 being rotated by the motor 7. The insert-side coupling means 8 being directly coupled to the machine-side coupling means 9 are caused to move in the same reciprocating manner. The insert-side coupling means 9 are connected (on both sides thereof) to one or more elongated members 27, such as a bar or rod, that reciprocates in the simultaneously past each row of medical instruments 3. This can be seen more clearly in figure 5. The elongated member 27 is provided with strategically positioned protrusions 28 arranged to engage and rotate associated sprocket wheels 29. An example of such an arrangement can be seen in figure 6. The sprocket wheels 29 are in turn connected to the operating elements 4 (in the form of wheels) via wheels 46. The protrusions 28 are positioned in such a way that the sprocket wheels 29 and thus the operating elements 4 are driven in a non-simultaneous manner. A slip coupling is provided with a multiwave spring 44 to prevent the exertion of excessive torque on the operating elements 4 and to allow the operating element 4 to be properly engaged when the operating element 4 itself is in an extreme position (i.e. the respective operating element 4 is not moved on the "forward" stroke of the reciprocating member 27, but on the "backward" stroke).

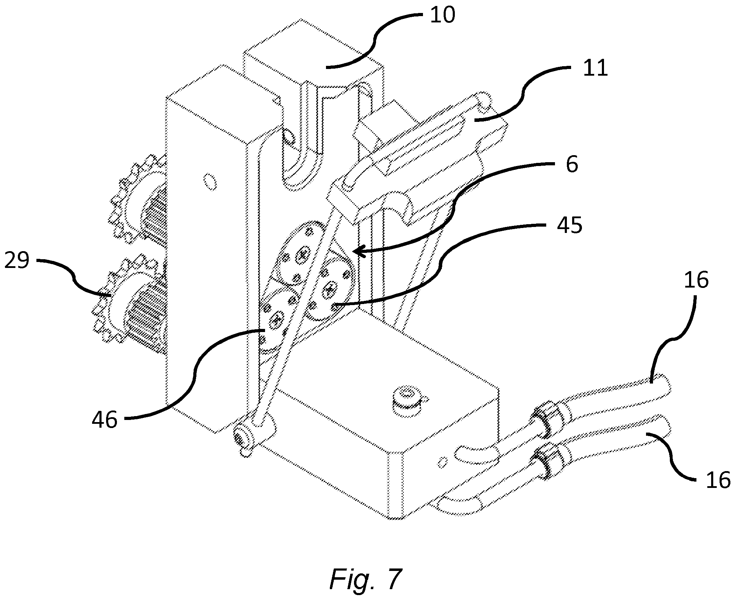

The wheels 46 of the operating means 6, such as three wheels, as shown, connect to the operating elements 4, often (also) in the form of wheels. The wheels 46 are provided with pins 45 provided with a spring mechanism (as can be more clearly seen in figure 7). The pins 45 are arranged for engaging corresponding recesses of the operating elements 4. Thus, when the wheels 46 are driven and rotated by the motor 7, they will automatically engage the operating elements 4.

The insert 1 as shown in figure 1 comprises three compartments, a central compartment 12 comprising the insert-side coupling means 8 and two side compartments 13 arranged for comprising the one or more medical instruments 3. Therein, the one or more medical instruments 3 are arranged in one or more rows 14 of six instruments 3 and the insert-side coupling means 8 are arranged for making a reciprocating movement past the operating means 6 of the one or more rows 14 of medical instruments 3.

The insert 1 further comprises connection means 15 for connecting one or more pressurized channels 16 to the one or more medical instruments 3. As stated before, preferably, per row 14 of mounting blocks 10 a cable (not shown) with an amount of pressure channels equal to the amount of medical instruments 3 in the row 14 is provided. The cable is preferably arranged for detachable connection to a cable connection point 30 on the insert/tray 1. At the cable connection point 30 the insert 1 is preferably provided with distribution means 31 providing individual pressurized channels 16 to each of the medical instruments 3, wherein each respective pressurized channel 16 connects to the respective pressurized channel comprised by the cable via the distribution means 31. In the embodiment as shown in figure 1, the cable comprises six pressurized channels (one per instrument 3).

Figure 7 shows a mounting block 10 having such pressurized channels 16 in more detail. The pins 45 can also be clearly seen. The pressurized channels 16 are preferably arranged for being monitored for pressure and flow by the device 2. The device may then give off a warning if a the pressure/flow in a channel 16 deviates from desired operational values, which may indicated clogging or leakage.

Figure 3 shows the device 2 for cleaning medical instruments 3 in more detail. The device 2 is arranged for receiving an aforementioned insert 1 at the top side 18 of the cleaning device 2. The top side 18 is provided with means for changing the height thereof to allow the operator to do his job in a convenient manner. Going back to figure 4, the cleaning device 2 comprises a motor 7 for driving the operating means 6, and machine-side coupling means 9 for being detachably coupled to the insert-side coupling means 8. The machine-side coupling means 9 are driveably coupled to the motor 7, such that, during use, the operating means 6 of the insert 1 can be driven by the motor. The reception space 17 is configured for containing a cleaning fluid for cleaning the one or more medical instruments 3 during use. The cleaning device 2, being in the form of a so-called "island", further comprises a chemistry cabinet 35, a switchbox cabinet 37 adjacent thereto and a technology cabinet 38 containing the motor, et cetera. The switchbox is preferably arranged on a sled or the like to facilitate removal/placement thereof in the switchbox cabinet 37. A touchscreen 36 is also provided to inform the operator of things like fluid (water) quantity, dosing of chemicals, temperature of the fluid in the reception space/ultrasonic tank 17, water pressure of the individual pressurized channels 16. A lid 33 is also shown for covering the reception space 17 during use. The lid 33 preferably automatically tilts when the insert 1 is lowered into or lifted out of the reception space 17. Via the touchscreen 36 the cleaning device 2 may provide warnings relating to for instance medical instruments 3 not being (properly) connected, medical instruments 3 being clogged, lack of compressed air pressure, lack of cold/warm water, lack of cleaning chemicals, et cetera. The cleaning chemicals are introduced from the chemistry cabinet 35 into the reception space/ultrasonic tank 17 via a flow meter. Preferably, the reception space 17 is heated, the heating for instance being controlled by a PT100.

The means for generating ultrasonic sound waves 19 are preferably configured to provide a degassing function as well as a so-called sweep function, wherein the sound waves are shifted with respect to each to prevent the formation of "knots". More preferably, bottom side 21 comprising the means for generating ultrasonic sound waves 19 is exchangeable/detachable to facilitate replacement in case one of the means for generating ultrasonic sound waves 19 becomes defect.

Furthermore, the cleaning chemicals are preferably arranged in cans in the chemistry cabinet 35. The cans are preferably arranged in a vessel or container capable of receiving the full contents of the cans, should such a can start leaking. In addition, the chemistry cabinet 35 is provided with means for discharging chemical vapours.

Preferably, the ultrasonic tank 17 is provided with both cold water supply means as well as hot water supply means to allow the cleaning fluid to quickly achieve its desired operating temperature.

Again referring back to figure 4, the reception space 17 is provided with means 19 for generating ultrasonic sound waves in the cleaning fluid, in the exemplary embodiment as shown comprising piezoelectric elements 20 arranged underneath a bottom side 21 of the reception space 17. The piezoelectric elements 20 are connected to the bottom side 21 in such a way, that the ultrasonic sound waves are transferred to the cleaning fluid by the bottom side 21. The recess 40 is comprised by a reciprocating element 41, which is arranged on a first spindle 26 driven by the motor 7. The motor 7 is connected to a second spindle 34 with end contact elements 42. The second spindle 34 and the end contact elements 42 can be used to configure the reciprocating movement of the system and to verify if the reciprocating movement is actually taking place.

Also, after the insert 1 is lowered into the reception space 17, the machine-side coupling means 9 are configured (when starting their reciprocating movement) to automatically "grab" or "engage" the insert-side coupling means 8 to facilitate automatic and proper coupling (as also explained with reference to figures 1 and 4).

The device 2 can also be provided with means for self-disinfection. During the ultrasonic cleaning operation the water reaches a temperature of 40°C, which allows bacteria to thrive. The means for self-disinfection (not shown) may therefore comprise a pumping mechanism and further heating elements to bring the water temperature to for instance 80°C, or at least to a temperature sufficient to kill micro-organisms such as bacteria, allowing for thermal disinfection of the water circuit. Another option could be to provide chemical disinfection by means of an appropriate pump/dosing mechanism and connection to the tank.

It should be clear that the description above is intended to illustrate the operation of preferred embodiments of the invention, and not to reduce the scope of protection of the invention. Starting from the above description, many embodiments will be conceivable to the skilled person within the inventive concept and scope of protection of the present invention.

### LIST OF REFERENCE NUMERALS

- 1.: Insert
- 2.: Device for cleaning medical instruments
- 3.: Medical instrument
- 4.: Operating element
- 5.: Moveable part of medical instrument
- 6.: Operating means for operating the medical instrument during cleaning
- 7.: Motor for driving the operating means
- 8.: Insert-side coupling means
- 9.: Machine-side coupling means
- 10.: Mounting block
- 11.: Clamp
- 12.: Central compartment
- 13.: Side compartment
- 14.: Row of mounting blocks/medical instruments
- 15.: Connection means
- 16.: Pressurized channel
- 17.: Reception space/ultrasonic tank
- 18.: Top side
- 19.: Means for generating ultrasonic sound waves
- 20.: Piezoelectric element
- 21.: Bottom side of reception space
- 22.: Holder part of endoscopic instrument
- 23.: Sidewalls delimiting lengthwise direction
- 24.: Lumen of endoscopic instrument
- 25.: Head/distal part of endoscopic instrument
- 26.: First spindle
- 27.: Elongated member
- 28.: Protrusion on elongated member
- 29.: Sprocket wheel
- 30.: Cable connection point
- 31.: Distribution means
- 32.: Suspension hook
- 33.: Lid
- 34.: Second spindle
- 35.: Chemistry cabinet
- 36.: Touchscreen
- 37.: Switch box cabinet
- 38.: Technology cabinet
- 39.: Pin
- 40.: Recess for engaging pin
- 41.: Reciprocating element comprising the recess
- 42.: End contact element
- 43.: Tilted walls/faces near recess
- 44.: Multiwave spring for use with slip coupling
- 45.: Pin with spring
- 46.: Wheel of operating means

## Claims

1. Insert (1) configured for being arranged in a device (2) for cleaning medical instruments, the insert being arranged for receiving one or more medical instruments (3) to be cleaned in the cleaning device, wherein the one or more medical instruments are provided with one or more operating elements (4) for moving one or more moveable parts (5) of the medical instruments, the insert comprising operating means (6) for operating the operating elements during cleaning, the operating means being arranged for connection with a motor (7) for driving the operating means during cleaning, **characterized in that** the cleaning device comprises the motor (7) for driving the operating means, wherein the insert is provided with insert-side coupling means (8) driveably coupled to the operating means, the insert-side coupling means being arranged for being detachably coupled to machine-side coupling means (9) comprised by the cleaning device, the machine-side coupling means being driveably coupled to the motor, such that, during use, the operating means of the insert can be driven by the motor.

2. Insert according to claim 1, comprising one or more mounting blocks (10) for mounting the one or more medical instruments, wherein the one or more mounting blocks preferably comprise one or more clamps (11) for properly connecting and positioning the one or more medical instruments with respect to the operating means during use.

3. Insert according to any one of the preceding claims, wherein the operating means for operating the operating elements during cleaning are configured for operating the operating elements in a non-simultaneous manner during use.

4. Insert according to any one of the preceding claims, wherein the insert is shaped like a tray.

5. Insert according to any one of the preceding claims, wherein the insert comprises three compartments, a central compartment (12), such as for comprising the insert-side coupling means, and two side compartments (13) arranged for comprising the one or more medical instruments.

6. Insert according to any one of the claims 2-5, wherein the one or more mounting blocks are arranged in one or more rows of mounting blocks, such that, during cleaning, the medical instruments are arranged in one or more rows (14) of instruments and a reciprocating member (27) is provided for reciprocating past the operating means of a row of mounting blocks.

7. Device (2) for cleaning medical instruments, arranged for receiving an insert according to any one of the preceding claims, wherein the cleaning device comprises the motor (7) for driving the operating means, wherein the insert is provided with insert-side coupling means (8) driveably coupled to the operating means, the insert-side coupling means being detachably coupled to machine-side coupling means (9) comprised by the cleaning device, the machine-side coupling means being driveably coupled to the motor, such that, during use, the operating means of the insert can be driven by the motor.

8. Device according to claim 7, wherein the cleaning device comprises a reception space (17) for receiving the insert at the top side (18) of the cleaning device.

9. Device according to claim 8, wherein the reception space is configured for containing a cleaning fluid for cleaning the one or more medical instruments during use and/or wherein the reception space is provided with means (19) for generating ultrasonic sound waves in the cleaning fluid.

10. Device according to claim 9, wherein the means for generating ultrasonic sound waves comprise piezoelectric elements (20).

11. Device according to claim 9 or 10, wherein the means for generating ultrasonic sound waves are arranged underneath a bottom side (21) of the reception space, the means being connected to the bottom side in such a way, that the ultrasonic sound waves are transferred to the cleaning fluid by the bottom side.

12. Assembly of an insert (1) according to any one of the claims 1-6 and a cleaning device (2) for cleaning medical instruments (3) according to any one of the claims 7-11, wherein the insert is arranged in the cleaning device, wherein the cleaning device comprises the motor (7) for driving the operating means, wherein the insert is provided with insert-side coupling means (8) driveably coupled to the operating means, the insert-side coupling means being detachably coupled to machine-side coupling means (9) comprised by the cleaning device, the machine-side coupling means being driveably coupled to the motor, such that, during use, the operating means of the insert can be driven by the motor.

## Patentansprüche

1. Einsatz (1), der dazu ausgelegt ist, in einer Vorrichtung (2) zur Reinigung von medizinischen Instrumenten angeordnet zu werden, wobei der Einsatz dazu angeordnet ist, ein oder mehrere in der Reinigungsvorrichtung zu reinigende medizinische Instrumente (3) aufzunehmen, wobei das eine oder die mehreren medizinischen Instrumente mit einem oder mehreren Betätigungselementen (4) zum Bewegen eines oder mehrerer beweglicher Teile (5) der medizinischen Instrumente versehen sind, wobei der Einsatz Betätigungseinrichtungen (6) zum Betätigen der Betätigungselemente während der Reinigung umfasst, wobei die Betätigungseinrichtungen zur Verbindung mit einem Motor (7) angeordnet sind, um die Betätigungseinrichtungen während der Reinigung anzutreiben, **dadurch gekennzeichnet, dass** die Reinigungsvorrichtung den Motor (7) zum Antreiben der Betätigungseinrichtungen umfasst, wobei der Einsatz mit einer einsatzseitigen Kopplungseinrichtung (8) versehen ist, die mit den Betätigungseinrichtungen antreibend gekoppelt ist, wobei die einsatzseitige Kopplungseinrichtung dazu angeordnet ist, mit einer in der Reinigungsvorrichtung enthaltenen maschinenseitigen Kopplungseinrichtung (9) lösbar gekoppelt zu werden, wobei die maschinenseitige Kopplungseinrichtung antreibend mit dem Motor gekoppelt ist, sodass die Betätigungseinrichtungen des Einsatzes während der Verwendung vom Motor angetrieben werden können.

2. Einsatz nach Anspruch 1, einen oder mehrere Montageblöcke (10) zur Montage des einen oder der mehreren medizinischen Instrumente umfassend, wobei der eine oder die mehreren Montageblöcke vorzugsweise eine oder mehrere Klemmen (11) umfassen, um das eine oder die mehreren medizinischen Instrumente in Bezug zu den Betätigungseinrichtungen während der Verwendung korrekt zu verbinden und anzuordnen.

3. Einsatz nach einem der vorstehenden Ansprüche, wobei die Betätigungseinrichtungen zum Betätigen der Betätigungselemente während der Reinigung dazu ausgelegt sind, die Betätigungselemente während der Verwendung nicht gleichzeitig zu betätigen.

4. Einsatz nach einem der vorstehenden Ansprüche, wobei der Einsatz schalenförmig ist.

5. Einsatz nach einem der vorstehenden Ansprüche, wobei der Einsatz drei Fächer umfasst, ein mittleres Fach (12), um zum Beispiel die einsatzseitige Kopplungseinrichtung zu umfassen, und zwei Seitenfächer (13), die dazu angeordnet sind, das eine oder die mehreren medizinischen Instrumente zu umfassen.

6. Einsatz nach einem der Ansprüche 2-5, wobei der eine oder die mehreren Montageblöcke in einer oder mehreren Reihen von Montageblöcken angeordnet sind, sodass die medizinischen Instrumente während der Reinigung in einer oder mehreren Reihen (14) von Instrumenten angeordnet sind, und ein Pendelelement (27) vorgesehen ist, um an den Betätigungseinrichtungen einer Reihe von Montageblöcken vorbei zu pendeln.

7. Vorrichtung (2) zur Reinigung medizinischer Instrumente, die dazu angeordnet ist, einen Einsatz nach einem der vorstehenden Ansprüche aufzunehmen, wobei die Reinigungsvorrichtung den Motor (7) zum Antreiben der Betätigungseinrichtungen umfasst, wobei der Einsatz mit einer einsatzseitigen Kopplungseinrichtung (8) versehen ist, die mit den Betätigungseinrichtungen antreibend gekoppelt ist, wobei die einsatzseitige Kopplungseinrichtung mit einer in der Reinigungsvorrichtung enthaltenen maschinenseitigen Kopplungseinrichtung (9) lösbar gekoppelt ist, wobei die maschinenseitige Kopplungseinrichtung mit dem Motor antreibend gekoppelt ist, sodass die Betätigungseinrichtungen des Einsatzes während der Verwendung vom Motor angetrieben werden können.

8. Vorrichtung nach Anspruch 7, wobei die Reinigungsvorrichtung einen Aufnahmeraum (17) zur Aufnahme des Einsatzes an der Oberseite (18) der Reinigungsvorrichtung umfasst.

9. Vorrichtung nach Anspruch 8, wobei der Aufnahmeraum dazu ausgelegt ist, ein Reinigungsfluid zu enthalten, um das eine oder die mehreren medizinischen Instrumente während der Verwendung zu reinigen, und/oder wobei der Aufnahmeraum mit einer Einrichtung (19) zum Erzeugen von Ultraschallwellen im Reinigungsfluid versehen ist.

10. Vorrichtung nach Anspruch 9, wobei die Einrichtung zum Erzeugen von Ultraschallwellen piezoelektrische Elemente (20) umfasst.

11. Vorrichtung nach Anspruch 9 oder 10, wobei die Einrichtung zum Erzeugen von Ultraschallwellen unter einer Unterseite (21) des Aufnahmeraums angeordnet ist, wobei die Einrichtung derart mit der Unterseite verbunden ist, dass die Ultraschallwellen über die Unterseite auf das Reinigungsfluid übertragen werden.

12. Anordnung eines Einsatzes (1) nach einem der Ansprüche 1-6 und einer Vorrichtung (2) zur Reinigung medizinischer Instrumente (3) nach einem der Ansprüche 7-11, wobei der Einsatz in der Reinigungsvorrichtung angeordnet ist, wobei die Reinigungsvorrichtung den Motor (7) zum Antreiben der Betätigungseinrichtungen umfasst, wobei der Einsatz mit einer einsatzseitigen Kopplungseinrichtung (8) versehen ist, die mit den Betätigungseinrichtungen antreibend gekoppelt ist, wobei die einsatzseitige Kopplungseinrichtung mit einer in der Reinigungsvorrichtung enthaltenen maschinenseitigen Kopplungseinrichtung (9) lösbar gekoppelt ist, wobei die maschinenseitige Kopplungseinrichtung mit dem Motor antreibend gekoppelt ist, sodass die Betätigungseinrichtungen des Einsatzes während der Verwendung vom Motor angetrieben werden können.

## Revendications

1. Insert (1) configuré pour être agencé dans un dispositif (2) de nettoyage d'instruments médicaux, l'insert étant agencé pour recevoir un instrument médical ou plusieurs instruments médicaux (3) à nettoyer dans le dispositif de nettoyage, où l'instrument médical ou les plusieurs instruments médicaux est/sont pourvu(s) d'un ou de plusieurs élément(s) d'actionnement (4) pour déplacer une ou plusieurs partie(s) mobile(s) (5) des instruments médicaux, l'insert comprenant des moyens d'actionnement (6) pour actionner les éléments d'actionnement pendant le nettoyage, les moyens d'actionnement étant agencés de manière à être reliés à un moteur (7) pour entraîner les moyens d'actionnement pendant le nettoyage, **caractérisé en ce que** le dispositif de nettoyage comprend le moteur (7) pour entraîner les moyens d'actionnement, où l'insert est pourvu de moyens de couplage côté insert (8) couplés de manière à pouvoir être entraînés aux moyens d'actionnement, les moyens de couplage côté insert étant agencés de manière à être couplés de manière amovible à des moyens de couplage côté machine (9) compris dans le dispositif de nettoyage, les moyens de couplage côté machine étant couplés de manière à pouvoir être entraînés au moteur, de sorte que, lors de l'utilisation, les moyens d'actionnement de l'insert puissent être entraînés par le moteur.

2. Insert selon la revendication 1, comprenant un ou plusieurs bloc(s) de montage (10) pour le montage de l'instrument médical ou des plusieurs instruments médicaux, où le ou les plusieurs bloc(s) de montage comprend/comprennent de préférence une ou plusieurs pinces (11) pour relier et positionner correctement l'instrument médical ou les plusieurs instruments médicaux par rapport aux moyens d'actionnement lors de l'utilisation.

3. Insert selon l'une quelconque des revendications précédentes, dans lequel les moyens d'actionnement pour actionner les éléments d'actionnement pendant le nettoyage sont configurés pour actionner les éléments d'actionnement de manière non simultanée lors de l'utilisation.

4. Insert selon l'une quelconque des revendications précédentes, dans lequel l'insert a la forme d'un plateau.

5. Insert selon l'une quelconque des revendications précédentes, dans lequel l'insert comprend trois compartiments, un compartiment central (12), tel que pour contenir les moyens de couplage côté insert, et deux compartiments latéraux (13) agencés pour contenir l'instrument médical ou les plusieurs instruments médicaux.

6. Insert selon l'une quelconque des revendications 2 à 5, dans lequel le ou les plusieurs bloc(s) de montage est/sont agencé(s) en une ou plusieurs rangée(s) de blocs de montage, de sorte que, lors du nettoyage, les instruments médicaux soient agencés en une ou plusieurs rangée(s) (14) d'instruments et un élément à mouvement de va-et-vient (27) est prévu pour effectuer un mouvement de va-et-vient au-delà des moyens d'actionnement d'une rangée de blocs de montage.

7. Dispositif (2) de nettoyage d'instruments médicaux, agencé pour recevoir un insert selon l'une quelconque des revendications précédentes, dans lequel le dispositif de nettoyage comprend le moteur (7) pour entraîner les moyens d'actionnement, où l'insert est pourvu de moyens de couplage côté insert (8) couplés de manière à pouvoir être entraînés aux moyens d'actionnement, les moyens de couplage côté insert étant couplés de manière amovible à des moyens de couplage côté machine (9) compris dans le dispositif de nettoyage, les moyens de couplage côté machine étant couplés de manière à pouvoir être entraînés au moteur, de sorte que, lors de l'utilisation, les moyens d'actionnement de l'insert puissent être entraînés par le moteur.

8. Dispositif selon la revendication 7, dans lequel le dispositif de nettoyage comprend un espace de réception (17) pour recevoir l'insert au niveau du côté supérieur (18) du dispositif de nettoyage.

9. Dispositif selon la revendication 8, dans lequel l'espace de réception est configuré pour contenir un fluide de nettoyage pour nettoyer l'instrument médical ou les plusieurs instruments médicaux lors de l'utilisation et/ou où l'espace de réception est pourvu de moyens (19) pour générer des ondes acoustiques ultrasonores dans le fluide de nettoyage.

10. Dispositif selon la revendication 9, dans lequel les moyens pour générer des ondes acoustiques ultrasonores comprennent des éléments piézoélectriques (20).

11. Dispositif selon la revendication 9 ou 10, dans lequel les moyens pour générer des ondes acoustiques ultrasonores sont agencés en dessous d'un côté inférieur (21) de l'espace de réception, les moyens étant reliés au côté inférieur de manière à ce que les ondes acoustiques ultrasonores soient transférées au fluide de nettoyage par le côté inférieur.

12. Ensemble d'un insert (1) selon l'une quelconque des revendications 1 à 6 et d'un dispositif de nettoyage (2) pour nettoyer des instruments médicaux (3) selon l'une quelconque des revendications 7 à 11, dans lequel l'insert est agencé dans le dispositif de nettoyage, où le dispositif de nettoyage comprend le moteur (7) pour entraîner les moyens d'actionnement, où l'insert est pourvu de moyens de couplage côté insert (8) couplés de manière à pouvoir être entraînés aux moyens d'actionnement, les moyens de couplage côté insert étant couplés de manière amovible à des moyens de couplage côté machine (9) compris dans le dispositif de nettoyage, les moyens de couplage côté machine étant couplés de manière à pouvoir être entraînés au moteur, de sorte que, lors de l'utilisation, les moyens d'actionnement de l'insert puissent être entraînés par le moteur.
